# EUROPEAN PATENT SPECIFICATION

(11) **EP 2 527 042 B1**
(45) Date of publication and mention of the grant of the patent: **17.01.2018**
(21) Application number: 12169382.4
(22) Date of filing: 24.05.2012
(51) Int. Cl.: B05B 7/04, B05B 7/00, B05B 7/12, B05B 13/02, A61L 31/08, A61M 5/32, B05D 5/08, B05D 1/02, B05B 1/30, B05B 7/10

(54) **Device and method for coating elongate objects**
Vorrichtung und Verfahren zum Beschichten länglicher Objekte
Dispositif et procédé de revêtement d'objets allongés

(30) Priority: 24.05.2011 US 201113114300
(43) Date of publication of application: 28.11.2012
(60) Divisional application: 16159480.9
(73) Proprietor: Nordson Corporation, Westlake, OH 44145-1119 (US)
(72) Inventor: Döker, Andreas, 82256 Fürstenfeldbruck (DE); Wanka, Thomas, 87600 Kaufbeuren (DE); Wilczek, Michael, 82110 Germering (DE)
(74) Representative: Eisenführ Speiser

(56) References cited:
- WO-A1-00/43570
- DE-A1- 2 213 984
- US-A- 4 106 519
- US-A- 4 221 339
- US-A- 5 743 963
- US-A- 6 117 480
- US-A1- 2005 238 829

## Description

### Technical Field

The present invention generally relates to the application of coatings onto exterior surfaces of objects and, more specifically, the coating of elongate objects such as needles.

### Background

Elongate objects are coated with material for various reasons. For example, hypodermic needles are often coated with lubricious or friction-reducing materials, such as silicone based oils. This provides a low friction outer surface of the needle for purposes of easing the introduction of the needle through the skin of a patient. Various methods and apparatus for applying the coating material to the needle have been used in the past. These include dipping methods in which the needles are dipped lengthwise into a bath containing silicone and solvent. This method presents difficulties and challenges related to handling the silicone and solvents, as well as the lengthy drying times involved, and the ventilation needs due to the use of the solvents.

Other methods that have been employed in the past involve various manners of spraying the coating material onto the needle. Typically, the spraying device will discharge the coating material in a single direction and the material will not flow around the entire exterior of the needle. For this reason, the needle and/or spray dispenser must be rotated to evenly distribute the coating material on the entire exterior surface of the needle. Alternatively, multiple spraying devices may be used to coat all sides of the needle. Either case involves increased complication and expense. The overspray or mist can also present problems in the environment around the equipment or require apparatus for containing the overspray. Due to the viscosity of the fluid coating material, it can also be difficult to achieve the required thin, uniform layer of coating material on the exterior surface of the needle.

US 2005/0238829 A1 discloses a method and a device for coating at least a portion of a medical device, wherein the method comprises the steps holding the medical device from an outer side surface and inserting a spray nozzle in a first opening accessing the interior of the medical device and spraying the coating on an inside surface of the medical appliance. In addition, US 5,743,963 A discloses an apparatus and a method for coating an object with a pair of rotating members each including a porous resilient roll which are mounted for counter-rotary movement.

It would therefore be desirable to provide a device and method for easily coating an exterior of an elongate workpiece, such as a needle, while addressing various challenges presented by past devices and methods.

### Summary

The present invention generally provides a device for coating an exterior of an elongate object. The device generally comprises a housing structure including an elongate coating chamber having first and second opposite ends. A port communicates with the first end for receiving the elongate object into the coating chamber and an outlet at the second end. The coating chamber has at least first and second sections. The first section is located closer to the outlet than the second section and the first section has a greater cross sectional area than the second section. The housing structure further includes an air supply passage and a coating material supply passage communicating with the elongate coating chamber. The pressurized air and coating material are adapted to enter the elongate chamber through the air supply passage and the coating material supply passage, respectively, to form a mist of the air and coating material. This mist is directed into the elongate chamber and generally toward the outlet while coating the exterior of the elongate object inserted into the elongate chamber through the port.

In a more specific embodiment, a mixing passage communicates with the air supply passage and the coating material supply passage. The mixing passage further communicates with the elongate chamber. The pressurized air and the coating material enter the mixing passage through the air supply passage and the coating material supply passage, respectively, and the mist of the air and coating material begins to form in the mixing passage before entering the elongate chamber.

A device constructed according to the exemplary embodiment further includes various additional features. For example, a valve comprised of a valve member and a valve seat selectively supplies the coating material to the elongate chamber. A ring shaped structure communicates between the air supply passage and the mixing passage. The ring shaped structure is configured to cause the pressurized air to enter the mixing passage with a swirling motion about a discharge location of the coating material into the mixing passage. The ring shaped further includes a central passage through which the coating material is directed into the mixing passage and into the air moving with the swirling motion. The device may further include a coating material injecting element including a tube with an outlet. The tube extends through the central passage with the outlet of the tube positioned in the mixing passage. The ring shaped structure more specifically includes a ring shaped wall surrounding an inner space and disposed around a central axis. The central passage extends along the central axis. The ring shaped wall further includes a plurality of air directing passages communicating between the air supply passage and the inner space so as to achieve the swirling motion around the central axis. The ring shaped structure further comprises a plurality of stand-off elements forming additional air passages between the stand-off elements and providing communication between the air supply passage and the mixing passage.

The elongate chamber is preferably configured with increasing diameter in a direction from the first end to the second end so that an increase in pressure is achieved in this direction within the chamber. The flow of the mist is generally along a central axis of the elongate chamber coaxial with the port at the first end. The mixing passage extends transverse, and more preferably perpendicular, to the elongate chamber.

The elongate object further comprises a hollow needle having opposite, open ends, such as a hypodermic syringe needle. The device further comprises a needle holder including an interior air space and a needle holding element configured to secure a first end of the needle in communication with the interior air space. The open second end of the needle extends outwardly from the needle holder for insertion through the port and into the elongate chamber. The interior air space is adapted to be pressurized with air to force air through the needle during a coating operation and prevent clogging of the open second end of the needle with the coating material.

In another aspect, a method is provided for coating an exterior surface of an elongate object with a coating material under use of the previously described device. The method generally includes holding the elongate object lengthwise in an elongate chamber having first and second opposite ends and an outlet at the second end. A mist is formed from a mixture of the air and the coating material and the exterior surface is coated with the coating material while directing the mist around the exterior surface of the elongate object and toward the outlet of the elongate chamber.

The method practiced according to an illustrative example includes various additional aspects and steps. For example, the pressurized air and the coating material are first mixed within a mixing passage oriented transverse to the elongate chamber prior to directing the air and the coating material into the elongate chamber as a mist. The mixing passage extends along an axis and the method further comprises swirling the air around the axis of the mixing passage at an air inlet to the mixing passage, and directing the coating material into the mixing passage along the central axis to mix with the swirling air. The method further comprises directing the air into the mixing passage with a Venturi effect. Holding the elongate object further comprises directing the object through a port at the first end of the elongate chamber and the method further comprises directing additional air through the port and adjacent the object toward the outlet of the elongate chamber. The elongate object further comprises a hollow needle with opposite, first and second open ends, and the method further comprises directing air through the first open end of the needle and out of the second open end positioned in the elongate chamber to prevent clogging of the first open end with the coating material. Directing the coating material into the elongate chamber further comprises actuating a valve to allow pressurized coating material to flow into the elongate chamber. The coating material may be a friction reducing material, such as a material containing silicone.

Various additional features and advantages of the invention will become more apparent to those of ordinary skill in the art upon review of the following detailed description of the illustrative embodiments taken in conjunction with the accompanying drawings.

### Brief Description of the Drawings

Fig. 1 is a perspective view of a device constructed in accordance with an illustrative embodiment of the invention.
Fig. 2 is a cross sectional view generally taken along line 2-2 of Fig. 1 and rotated 90° for purposes of easier explanation.
Fig. 2A is an enlarged view of the portion marked "2A" in Fig. 2.
Fig. 3 is a perspective view of the ring shaped structure used to direct the air flow within the device of Fig. 1.
Fig. 4 is a cross sectional view taken generally along line 4-4 of Fig. 3.

### Detailed Description of the Illustrative Embodiments

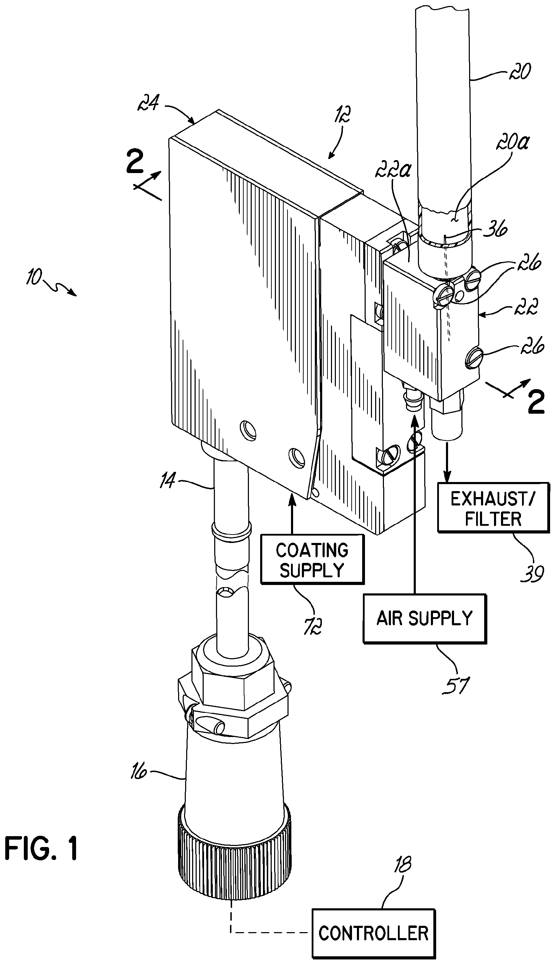

Fig. 1 illustrates a device 10 generally comprising a housing structure 12 coupled with a conduit 14 suitable for containing control and electrical supply wiring (not shown). An electrical coupler 16 is provided and adapted to couple the device 10 to a controller 18 for operating the device 10. A needle holder 20 is provided for introducing a needle to be coated, as further discussed below, into the housing structure 12. More specifically, the needle holder 20 is adapted to abut an outside face 22a of a first sub-housing 22 coupled to a second sub-housing 24 of the housing structure 12 by fasteners 26.

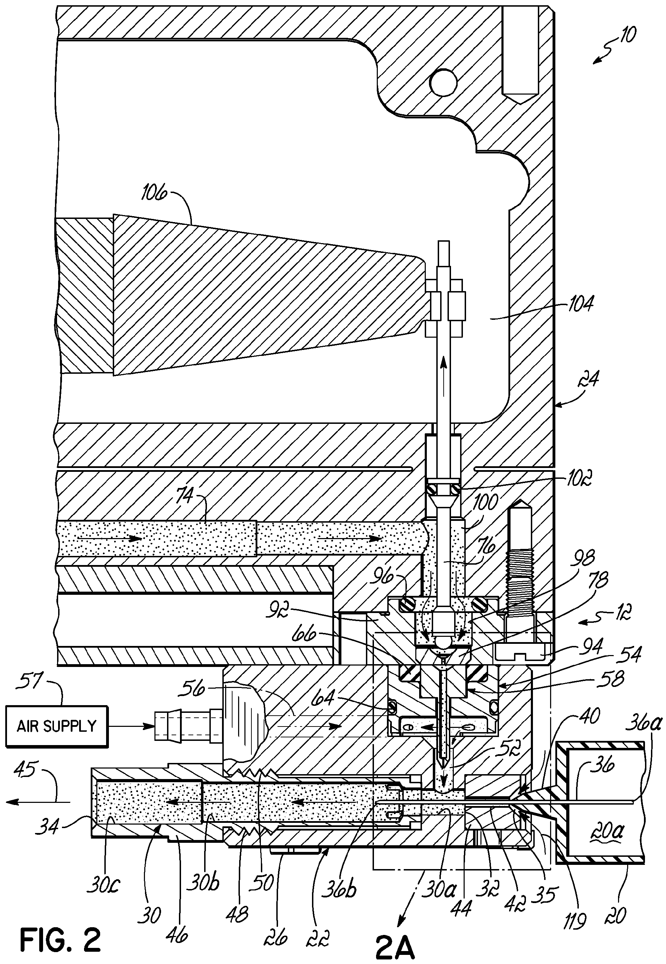

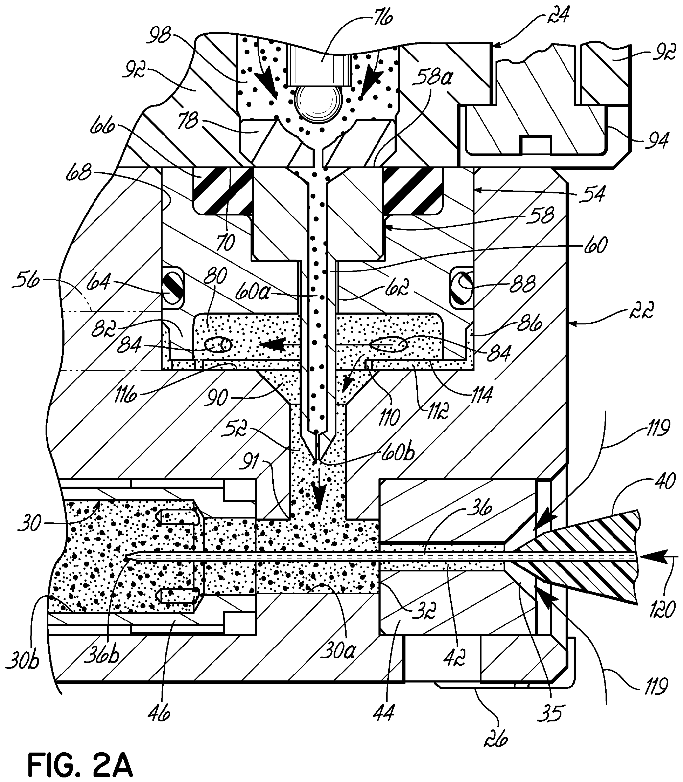

Referring to Figs. 2 and 2A, the first sub-housing 22 more specifically comprises an elongate chamber 30 having first and second opposite ends 32, 34. A port 35 at the first end 32 receives a needle 36 for coating purposes. The second end 34 defines an outlet. As shown in Fig. 1, the second end is fluidly coupled to an exhaust 39 including a filter. The needle holder 20 is mounted for movement in a manner (not shown) that allows for repeatable introduction of successive needles 36 into the chamber 30 during a manufacturing operation. The needle holder 20 includes an interior air space 20a and a needle holding element 40. The needle holding element 40 grips the needle 36 with friction or in any other suitable manner such that an open first end 36a communicates with the interior air space 20a and an open second end 36b is positionable within the elongate chamber 30. The needle 36 extends through the port 35 preferably along the central axis of the port 35 and the elongate chamber 30. The needle 36 extends through a passage 42 in an insert 44 within the first sub-housing 22 and the port 35 more specifically comprises a conically shaped opening in the insert 44. As will be more specifically described below, air is drawn in through the conically shaped port 35 into the passage 42 of the insert 44 and, finally, into the elongate chamber 30. The elongate chamber 30 comprises cylindrical chamber portions 30a, 30b, 30c of successively larger diameter for creating an increasing pressure as pressurized air flows from the first end 32 toward the second end 34 in the direction of the arrows 45. At least a portion of the elongate chamber 30 may be formed by the interior of a tubular member 46 that is removably attached to the first sub-housing 22 by respective threads 48, 50.

A mixing passage 52 in the first sub-housing 22 extends transverse and, more specifically, perpendicular to the elongate chamber 30. This mixing passage 52 communicates directly with the smallest diameter chamber portion 30a adjacent the insert 44 and needle port 35. As best shown in Fig. 2A, the first sub-housing 22 further includes a ring shaped structure 54 for receiving pressurized air from an air supply passage 56. The air supply passage 56 is coupled with a source 57 of pressurized air. The pressure of the air is preferably in the range of one to four bar. The ring shaped structure 54 further receives a coating material injecting element 58 including a tube 60 with a passage 60a and an outlet 60b extending through a central passage 62 of the ring shaped structure 54. The ring shaped structure 54 more specifically comprises a ring sealed by respective O-rings 64, 66 within a recess 68 of the first sub-housing 22 and against a surface 70 of the second sub-housing 24.

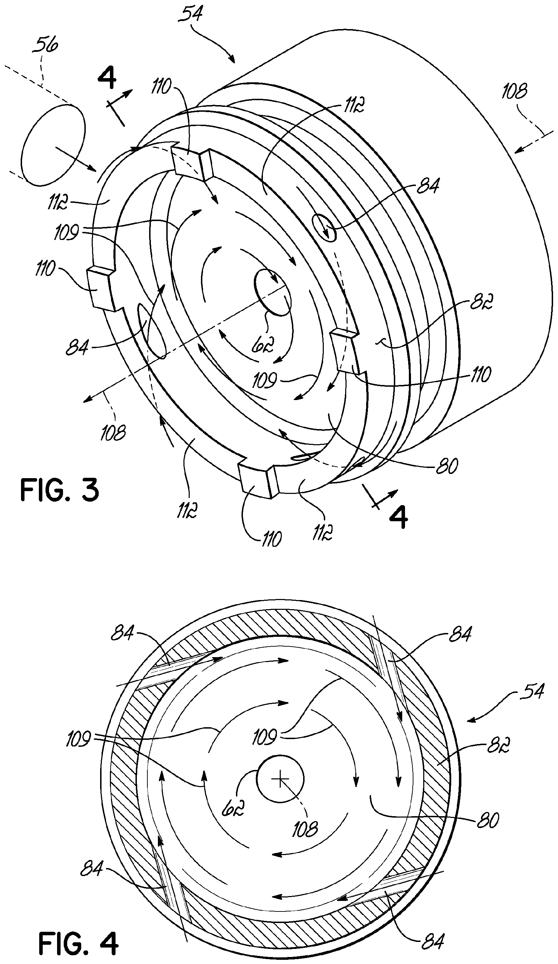

The coating material is provided from a suitable pressurized supply 72 (Fig. 1). In the case of coating a hypodermic syringe needle, for example, the coating materials are preferably silicone oils of various viscosity ranging, for example, from the viscosity of water to 12,500 mPas. Most applications currently contemplated will utilize a viscosity of 1000 - 3000 mPas. The coating material is directed through a supply passage 74 (Fig. 2) and ultimately into the central passage 60a of the tube 60 and is injected into the mixing passage 52 when a valve element 76 positioned in the second sub-housing 24 is opened relative to a valve seat element 78 abutting an inlet end 58a of the coating material injecting element 58. As will be described further in connection with Figs. 3 and 4, the ring shaped structure 54 receives and directs the air in a swirling motion within an inner space 80 defined by a ring shaped or annular wall 82. The ring shaped wall 82 includes a plurality of air directing passages 84 communicating with the air supply passage 56 through an annular air space 86 formed between the ring shaped wall 82 and the wall 88 of the recess 68. The swirling air within an inner space 80 is directed downwardly into a generally cone-shaped or converging passage 90 surrounding the tube 60 of the coating material injecting element 58 and then into the mixing passage 52. Due to the constriction formed by the converging passage 90, the air is directed into the mixing passage with a Venturi effect in addition to the swirling motion. The air then mixes with the injected coating material to form a mixture in the form of a mist as the air and coating material enter the elongate chamber 30 through a connecting port 91.

More specifically referring to Fig. 2, the valve seat element 78 is carried on a valve component 92 affixed to a main part of the second sub-housing 24 by threaded fasteners 94 (only one shown). The valve component 92 is mounted in sealing engagement with the main part of the second sub-housing 24 through the use of an O-ring 96. A passage 98 of the valve component 92 communicates with a passage 100 in the second sub-housing 24 containing the reciprocating valve element or valve stem 76. The passage 100 communicates with the coating material supply passage 74 and, ultimately, with the coating material supply 72 (Fig. 1). The valve stem 76 includes an additional dynamic seal 102 engaged with the internal wall defining the passage 100 to prevent the coating material from leaking into the interior space 104 of the second sub-housing 24 which contains valve stem actuating components, including an actuating arm 106. The valve stem 76 and actuating arm 106 may be actuated in any suitable manner such as by using a conventional piezoelectric actuator (not shown).

Referring to Fig. 2A in combination with Figs. 3 and 4, the ring shaped structure 54 includes the ring shaped wall 82 surrounding the inner space 80 and the wall 82 is spaced around a central axis 108. The central passage 62 extends in a coaxial fashion around the central axis 108 and the ring shaped wall 82 further includes the plurality of air directing passages 84 communicating with the annular space 86 (Fig. 2A) which, in turn, communicates with the pressurized air supply passage 56. The air directing passages 84 are formed in a generally tangential manner relative to the cylindrical shape of the inner space 80 as best illustrated in Fig. 4 such that air entering the inner space 80 travels with a swirling motion as shown by the arrows 109. A plurality of stand-off elements 110 extends from a lower end of the ring shaped structure 54 and provide for additional air passages or paths 112 (Fig. 2A) between the stand-off elements 110 and between lower surfaces 114 of the ring shaped structure 54 and the bottom wall 116 (Fig. 2A) of the recess 68, as shown in Fig. 2A. Therefore, air will enter an annular space 86 from the air supply passage 56 and, from the annular space 86, pass through the air directing passages 84 into the inner space 80, and also pass between the stand-off elements 110 through passages 112. The air passing through the air directing passages 84 will cause a swirling air motion within the inner space 80 and this air will be directed downwardly to combine with the air passing between the stand-off elements 110 and then enter the inlet 90 of the air mixing passage 52 with a swirling motion and Venturi effect, i.e., reduced pressure and increased velocity. This mixing action will begin to form a mist with the coating material being injected through the outlet 60b of the tube 60 along the central axis 108 of the ring shaped structure 54 and the coaxial central axis of the mixing passage 52. The flow within the elongate chamber 30 will draw additional air into the port 35 as shown by arrows 119 and through the passage 42 of the insert 44 (see Figs. 2 and 2A) to prevent any coating material from traveling in the opposite direction through the port 35. At the same time, pressurized air in the interior air space 20a will travel as illustrated by the arrow 120 in Fig. 2A through the open first end 36a of the needle 36 and out of the open second end 36b of the needle 36 to prevent the second end 36b of the needle 36 from being clogged by coating material in the mist. The controller 18 (Fig. 1) will operate the actuating arm 106 to open and close the valve stem 76, and operate the pressurized air in an on/off fashion as necessary to successively coat multiple needles 36 in serial fashion as they are introduced one-by-one into the elongate chamber 30 by the same or different needle holders 20.

The first sub-housing 22 may be removed from the second sub-housing 24 for repair and replacement purposes. More specifically, components contained in the first sub-housing 22 may become contaminated due to the environmental air that is being suctioned into the passages of those components and mixed with the coating material. On the other hand, the components and passages associated with the second sub-housing 24 need much less service during regular use. Thus, the second sub-housing 24 and its associated components may remain mounted while the first sub-housing 22 and its attached components are removed from the second sub-housing by removing fasteners 26. The components 44, 46, 54, 58 associated with the first sub-housing 22 may be easily disassembled for cleaning purposes. In addition, the modular nature of the first sub-housing 22 allows different application requirements to be accommodated, such as size changes to accommodate different elongate objects, and adjustments to the type and/or viscosity of coating material. In addition, the injecting element 58 may be replaced to accommodate a coating material having a different viscosity. Finally, the insert 44 receiving the elongate object 36 may be modified for particular applications, such as coating operations of syringe tips with various configurations.

### Abstract

### DEVICE AND METHOD FOR COATING ELONGATE OBJECTS

A device for coating an exterior of an elongate object includes a housing structure with an elongate chamber having first and second opposite ends. A port communicates with the first end for receiving the elongate object and an outlet is located at the second end. An air supply passage and a coating material supply passage communicate with the elongate chamber. Pressurized air and coating material are adapted to enter the elongate chamber through the air supply passage and the coating material supply passage, respectively, to form a mist in the elongate chamber moving toward the outlet while coating the exterior of the object. A method of coating an exterior surface of the elongate object with the coating material includes holding the elongate object lengthwise in the elongate chamber, mixing the pressurized air and the coating material to form a mist, and coating the exterior surface while directing the mist around the exterior surface and toward the outlet of the elongate chamber.

While the present invention has been illustrated by a description of various preferred embodiments and while these embodiments have been described in some detail, it is not the intention of the Applicants to restrict or in any way limit the scope of the appended claims to such detail. Additional advantages and modifications will readily appear to those skilled in the art. The various features of the invention may be used alone or in any combination depending on the needs and preferences of the user. This has been a description of the present invention, along with the preferred methods of practicing the present invention as currently known. However, the invention itself should only be defined by the appended claims. What is claimed is:

## Claims

1. A device (10) for coating an exterior of an elongate object, comprising:
a housing structure (12) including an elongate coating chamber (30) having first and second opposite ends (32, 34), a port (35) communicating with said first end (32, 34) for receiving the elongate object into the coating chamber (30) and an outlet at said second end, said coating chamber (30) having at least first and second sections, said first section located closer to said outlet than said second section and said first section having a greater cross sectional area than said second section, the housing structure (12) further including an air supply passage (56) and a coating material supply passage (74) communicating with said elongate coating chamber (30);
wherein pressurized air and coating material are adapted to enter said elongate chamber (30) through said air supply passage (56) and said coating material supply passage (74), respectively, to form a mist of the air and coating material that is directed into said elongate chamber (30) and generally toward said outlet while coating the exterior of the elongate object inserted into said elongate chamber (30) through said port (35).

2. The device of claim 1, further comprising:
a mixing passage (52) communicating with said air supply passage (56) and said coating material supply passage (74), said mixing passage (52) further communicating with said elongate chamber (30), wherein pressurized air and coating material are adapted to enter said mixing passage (30) through said air supply passage (56) and said coating material supply passage (74), respectively, to form the mist of the air and coating material that is directed into said elongate chamber (30) and generally toward said outlet while coating the exterior of the object inserted into said elongate chamber (30) through said port (35).

3. The device of claim 2, wherein said elongate chamber (30) includes a central axis coaxial with said port (35), and said mixing passage (52) extends transverse to said elongate chamber (30).

4. The device of claim 1, further comprising:
a ring shaped structure (54) communicating between said air supply passage (56) and said elongate chamber (30), said ring shaped structure (54) configured to cause a swirling motion of the pressurized air, said ring shaped structure (54) further including a central passage (62) through which the coating material is adapted to be directed into the air moving with the swirling motion to thereby form the mist.

5. The device of claim 4, further comprising a coating material injecting element (58) including a tube (60) with an outlet (60b), said tube (60) extending through said central passage (62).

6. The device of claim 5, wherein said ring shaped structure (54) further comprises a ring shaped wall (82) surrounding an inner space (80) and disposed around a central axis (108), and wherein said central passage (62) extends along the central axis (108), said ring shaped wall (82) further including a plurality of air directing passages (84) communicating between said air supply passage (56) and said inner space (80) and directing the pressurized air into said inner space (80) in the swirling motion around said central axis (108), and/or said ring shaped structure (54) further comprises a plurality of stand-off elements (110) forming additional air passages communicating between said air supply passage (56) and said elongate chamber (30).

7. The device of claim 1, further comprising:
a valve member (76) and a valve seat (78) mounted in said coating material supply passage (74), said valve member (76) movable with respect to said valve seat (78) to selectively supply the coating material to the elongate chamber (30).

8. The device of claim 7, wherein said housing structure (12) further comprises a first sub-housing (22) and a second sub-housing (24), said first sub-housing (22) being removably coupled to said second sub-housing (24) and containing said elongate coating chamber (30), and said second sub-housing (24) containing said valve member (76) and said valve seat (78).

9. The device of claim 1, wherein the elongate object further comprises a hollow needle, and further comprising:
a needle holder (20) including an interior air space (20a) and a needle holding element (40) configured to secure a first end (36a) of the needle (36) in communication with the interior air space (20a) while an open second end (36b) of the needle (36) extends outwardly from said needle holder (20) for insertion through said port (35) and into said elongate chamber (30), wherein said interior air space (20a) is adapted to be pressurized with air to force air through the needle (36) and prevent clogging of the open second end (36b) of the needle (36) with the coating material.

10. The device of claim 1, wherein said port (35) is configured to allow additional air to be drawn into said elongate chamber (30) as the mist is coating the exterior of the elongate object.

11. A method of coating an exterior surface of an elongate object with a coating material under use of a device according to claim 1, comprising:
holding the elongate object lengthwise in an elongate chamber (30) having first and second opposite ends (32, 34) and an outlet at the second end (34),
mixing pressurized air and the coating material to form a mist; and coating the exterior surface with the coating material while directing the mist around the exterior surface and toward the outlet of the elongate chamber (30).

12. The method of claim 11, further comprising:
mixing the pressurized air and the coating material in a mixing passage (52) oriented transverse to the elongate chamber (30) prior to directing the air and the coating material into the elongate chamber (30),
wherein preferably the mixing passage (52) extends along an axis, and the method further comprises:
directing the air into the mixing passage (52) with a Venturi effect.

13. The method of claim 11, wherein holding the elongate object further comprises directing the object through a port (35) communicating with the first end (32) of the elongate chamber (30), and the method further comprises:
directing additional air through the port (35) and adjacent the object toward the outlet of the elongate chamber (30).

14. The method of claim 11, wherein the elongate object further comprises a hollow needle with opposite, first and second open ends (36a, 36b), and the method further comprises:
locating the second open end (36b) in the elongate chamber (30); and directing air through the first open end (36a) of the needle (36) and out of the second open end (36b) to prevent clogging of the second open end (36b) with the coating material.

15. The method of claim 11, wherein directing the coating material further comprises:
actuating a valve to allow pressurized coating material to flow into the elongate chamber (30).

16. The method of claim 11, wherein the coating material comprises a friction reducing material.

17. The method of claim 11, wherein directing the mist toward the outlet further comprises:
directing the mist through sections of the chamber (30) having increasing diameter.

## Patentansprüche

1. Eine Vorrichtung zum Beschichten einer Außenseite eines länglichen Gegenstandes, aufweisend:
eine Gehäusestruktur (12) umfassend eine längliche Beschichtungskammer (30) mit einem ersten und einem zweiten gegenüberliegenden Ende (32, 34), einem Anschluss (35), der mit dem ersten Ende zum Aufnehmen des länglichen Gegenstandes in der Beschichtungskammer (30) in Verbindung steht, und mit einem Auslass an dem zweiten Ende, wobei die Beschichtungskammer (30) mindestens einen ersten und einen zweiten Abschnitt aufweist, wobei der erste Abschnitt näher an dem Auslass angeordnet ist als der zweite Abschnitt und wobei der erste Abschnitt einen größeren Querschnitt aufweist als der zweite Abschnitt, wobei die Gehäusestruktur (12) ferner einen Luftversorgungs-Durchgang (56) und einen Beschichtungsmaterial-Versorgungsdurchgang (74) umfasst, welche mit der länglichen Beschichtungskammer (30) in Verbindung stehen;
wobei Druckluft und Beschichtungsmaterial dazu eingerichtet sind, in die längliche Kammer (30) durch den Luftversorgungs-Durchgangs (56) bzw. den Beschichtungsmaterial-Versorgungsdurchgang (74) einzutreten, um einen Nebel aus Luft und Beschichtungsmaterial zu bilden, der in die längliche Beschichtungskammer (30) und im Allgemeinen in Richtung des Auslasses gerichtet ist, während des Beschichtens der Außenseite des länglichen Gegenstandes, der in die längliche Kammer (30) durch den Anschluss (35) eingesetzt ist.

2. Die Vorrichtung nach Anspruch 1, ferner ausweisend:
einen Mischdurchgang (52), der mit dem Luftversorgungs-Durchgang (56) und dem Beschichtungsmaterial-Versorgungsdurchgang (74) in Verbindung steht, wobei der Mischdurchgang (52) ferner mit der länglichen Kammer (30) in Verbindung steht, wobei Druckluft und Beschichtungsmaterial dazu eingerichtet sind in den Mischdurchgang (52) durch den Luftversorgungs-Durchgang (56) bzw. den Beschichtungsmaterial-Versorgungsdurchgang (74) einzutreten, um den Nebel aus Luft und Beschichtungsmaterial zu bilden, der in die längliche Beschichtungskammer (30) und im Allgemeinen in Richtung des Auslasses gerichtet ist, während des Beschichtens der Außenseite des länglichen Gegenstandes, der in die längliche Kammer (30) durch den Anschluss (35) eingesetzt ist.

3. Die Vorrichtung nach Anspruch 2, wobei die längliche Kammer (30) eine Mittenachse aufweist, die koaxial zu der Öffnung (35) ist, und wobei sich der Mischdurchgang (52) quer zur länglichen Kammer (30) erstreckt.

4. Die Vorrichtung nach Anspruch 1, ferner aufweisend:
eine ringförmige Struktur (54), die zwischen dem Luftversorgungs-Durchgang (56) und der länglichen Kammer (30) in Verbindung steht, wobei die ringförmige Struktur (54) dazu konfiguriert ist, um eine Wirbelbewegung der Druckluft zu bewirken, wobei die ringförmige Struktur ferner einen zentralen Durchgang (62) aufweist, durch welchen das Beschichtungsmaterial eingerichtet ist, in die sich mit der Wirbelbewegung bewegende Luft gerichtet zu werden, um dadurch den Nebel zu bilden.

5. Die Vorrichtung nach Anspruch 4 ferner aufweisend ein Beschichtungsmaterial-Einspritzelement (58), dass ein Rohr (60) mit einem Auslass (60b) umfasst, wobei das Rohr (60) sich durch den zentralen Durchgang (62) erstreckt.

6. Die Vorrichtung nach Anspruch 5, wobei die ringförmige Struktur (54) ferner eine ringförmige Wand (82) aufweist, die einen Innenraum (80) umgibt und um eine zentrale Achse (108) herum angeordnet ist, und wobei der zentrale Durchgang (62) sich entlang der zentralen Achse (108) erstreckt, wobei die ringförmige Wand (82) ferner eine Vielzahl von Luftleitdurchgängen (84) aufweist, die zwischen den Luftversorgungs-Durchgängen (56) und dem Innenraum (80) kommunizieren und die Druckluft in den Innenraum (80) mit der Wirbelbewegung um die zentrale Achse (108) einleiten, und/oder wobei die ringförmige Struktur (54) ferner eine Vielzahl von Abstandselementen (110) aufweist, die zusätzliche Luftkanäle bilden, die zwischen den Luftversorgungs-Durchgang (56) und der länglichen Kammer (30) kommunizieren.

7. Die Vorrichtung nach Anspruch 1, ferner aufweisend, ein Ventilelement (76) und ein Ventilsitz (78), die in dem Beschichtungsmaterial-Versorgungsdurchgang (74) montiert sind, wobei das Ventilelement (76) im Bezug auf den Ventilsitz (78) bewegbar ist, um selektiv das Beschichtungsmaterial zu der länglichen Kammer (30) zu leiten.

8. Die Vorrichtung nach Anspruch 7, wobei die Gehäusestruktur (12) ferner ein erstes Teilgehäuse (22) und ein zweites Teilgehäuse (24) aufweist, wobei das erste Teilgehäuse (22) lösbar mit dem zweiten Teilgehäuse (24) verbunden ist und die längliche Beschichtungskammer (30) enthält, und wobei das zweite Teilgehäuse (24) das Ventilelement und den Ventilsitz (78) enthält.

9. Die Vorrichtung nach Anspruch 1, wobei der länglichen Gegenstand ferner eine hohle Nadel aufweist, und ferner aufweisend:
einen Nadelhalter (20) umfassend einen inneren Luftraum (20a) und ein Nadel-Halteelement (40), das dazu konfiguriert ist, um ein erstes Ende (36a) der Nadel (36) zu sichern, das mit dem inneren Luftraum (20a) in Verbindung steht, während ein offenes zweites Ende (36b) der Nadel (36) sich von dem Nadelhalter (20) nach außen zum Einführen durch die Öffnung (35) in die längliche Kammer (30) erstreckt, wobei der innere Luftraum (20a) dazu eingerichtet ist, mit Luftdruck beaufschlagt zu werden um Luft durch die Nadel (36) zu zwingen und ein Verstopfen des offenen zweiten Endes (36b) der Nadel (36) mit Beschichtungsmaterial zu verhindern.

10. Die Vorrichtung nach Anspruch 1, wobei der Anschluss (35) dazu konfiguriert ist, um zu ermöglichen, dass zusätzlich Luft in die längliche Kammer (30) gesogen wird, wenn der Nebel die Außenseite des länglichen Gegenstandes beschichtet.

11. Ein Verfahren zum Beschichten einer äußeren Oberfläche eines länglichen Gegenstandes mit einem Beschichtungsmaterial unter Verwendung einer Vorrichtung nach Anspruch 1, aufweisend:
Halten des länglichen Gegenstandes in Längsrichtung in einer länglichen Kammer (30), die ein erstes und ein zweites entgegengesetztes Ende (32, 34) und einen Auslass am zweiten Ende (34) aufweist,
Mischen von Druckluft und dem Beschichtungsmaterial, um einen Nebel zu bilden; und
Beschichten der äußeren Oberfläche mit dem Beschichtungsmaterial während der Nebel um die äußere Oberfläche und in Richtung des Auslasses der länglichen Kammer (30) gerichtet wird.

12. Das Verfahren nach Anspruch 11, ferner aufweisend:
Mischen der Druckluft und des Beschichtungsmateriales in einem Mischdurchgang (52), der quer zur länglichen Kammer (30) ausgerichtet ist, bevor die Luft und das Beschichtungsmaterial in die längliche Kammer (30) eingeleitet werden,
wobei bevorzugt der Mischdurchgang (52) sich entlang einer Achse erstreckt, und das Verfahren ferner aufweist:
Einleiten der Luft in den Mischdurchgang (52) mit einem Venturieffekt.

13. Das Verfahren nach Anspruch 11, wobei das Halten des länglichen Gegenstandes ferner das Führen des Gegenstandes durch einen Anschluss (35) umfasst, die mit dem ersten Ende (32) der länglichen Kammer (30) in Verbindung steht, und das Verfahren ferner aufweist,
Einleiten zusätzlicher Luft durch den Anschluss (35) und angrenzend an den Gegenstand in Richtung des Auslasses in der länglichen Kammer (30).

14. Das Verfahren nach Anspruch 11, wobei der längliche Gegenstand ferner eine hohle Nadel mit entgegengesetzten ersten und zweiten offenen Enden (36a, 36b) aufweist, und das Verfahren ferner aufweist:
Anordnen des zweiten offenen Endes (36b) in der länglichen Kammer (30); und
Einleiten von Luft durch die erste Öffnung (36a) der Nadel (36) und aus dem zweiten offenen Ende (36b), um ein Verstopfen des zweiten offenen Endes (36b) mit dem Beschichtungsmaterial zu verhindern.

15. Das Verfahren nach Anspruch 11, wobei das Einleiten des Beschichtungsmateriales ferner aufweist:
Betätigen eines Ventils, um zu ermöglichen, dass unter Druck stehende Beschichtungsmaterial in die längliche Kammer (30) einströmt.

16. Das Verfahren nach Anspruch 11, wobei das Beschichtungsmaterial ein reibungsreduzierendes Material aufweist.

17. Das Verfahren nach Anspruch 11, wobei das Richten des Nebels in Richtung des Auslasses ferner aufweist:
Richten des Nebels durch Abschnitte der Kammer (30) mit zunehmendem Durchmesser.

## Revendications

1. Dispositif (10) de revêtement d'un extérieur d'un objet allongé, comprenant :
une structure de logement (12) comprenant une chambre de revêtement allongée (30) ayant des première et deuxième extrémités opposées (32, 34), un orifice (35) communiquant avec ladite première extrémité (32, 34) pour recevoir l'objet allongé dans la chambre de revêtement (30) et une sortie à ladite deuxième extrémité, ladite chambre de revêtement (30) ayant au moins des première et deuxième sections, ladite première section étant située plus proche de ladite sortie que ladite deuxième section et ladite première section ayant une plus grande surface de section transversale que ladite deuxième section, la structure de logement (12) comprenant en outre un passage de distribution d'air (56) et un passage de distribution de matériau de revêtement (74) communiquant avec ladite chambre de revêtement allongée (30) ;
dans lequel de l'air comprimé et du matériau de revêtement sont adaptés pour entrer dans ladite chambre allongée (30) à travers ledit passage de distribution d'air (56) et ledit passage de distribution de matériau de revêtement (74), respectivement, pour former un brouillard d'air et de matériau de revêtement qui est dirigé dans ladite chambre allongée (30) et généralement vers ladite sortie tout en revêtant l'extérieur de l'objet allongé inséré dans ladite chambre allongée (30) à travers ledit orifice (35).

2. Dispositif selon la revendication 1, comprenant en outre :
un passage de mélange (52) communiquant avec ledit passage de distribution d'air (56) et ledit passage de distribution de matériau de revêtement (74), ledit passage de mélange (52) communiquant en outre avec ladite chambre allongée (30), dans lequel de l'air comprimé et du matériau de revêtement sont adaptés pour entrer dans ledit passage de mélange (30) à travers ledit passage de distribution d'air (56) et ledit passage de distribution de matériau de revêtement (74), respectivement, pour former le brouillard d'air et de matériau de revêtement qui est dirigé dans ladite chambre allongée (30) et généralement vers ladite sortie tout en revêtant l'extérieur de l'objet inséré dans ladite chambre allongée (30) à travers ledit orifice (35).

3. Dispositif selon la revendication 2, dans lequel ladite chambre allongée (30) comprend un axe central coaxial avec ledit orifice (35), et ledit passage de mélange (52) s'étend transversalement à ladite chambre allongée (30).

4. Dispositif selon la revendication 1, comprenant en outre :
une structure en forme d'anneau (54) communiquant entre ledit passage de disribution d'air (56) et ladite chambre allongée (30), ladite structure en forme d'anneau (54) étant configurée pour provoquer un mouvement de tourbillonnement de l'air comprimé, ladite structure en forme d'anneau (54) comprenant en outre un passage central (62) à travers lequel le matériau de revêtement est adapté pour être dirigé dans l'air mû dudit mouvement de tourbillonnement pour former ce faisant le brouillard.

5. Dispositif selon la revendication 4, comprenant en outre un élément d'injection de matériau de revêtement (58) comprenant un tube (60) avec une sortie (60b), ledit tube (60) s'étendant à travers ledit passage central (62).

6. Dispositif selon la revendication 5, dans lequel ladite structure en forme d'anneau (54) comprend en outre une paroi en forme d'anneau (82) entourant un espace intérieur (80) et disposée autour d'un axe central (108), et dans lequel ledit passage central (62) s'étend le long de l'axe central (108), ladite paroi en forme d'anneau (82) comprenant en outre une pluralité de passages de direction d'air (84) communiquant entre ledit passage de distribution d'air (56) et ledit espace intérieur (80) et dirigeant l'air comprimé dans ledit espace intérieur (80) dans le mouvement de tourbillonnement autour dudit axe central (108), et/ou ladite structure en forme d'anneau (54) comprenant en outre une pluralité d'éléments d'éloignement (110) formant des passages d'air supplémentaires communiquant entre ledit passage de distribution d'air (56) et ladite chambre allongée (30).

7. Dispositif selon la revendication 1, comprenant en outre :
un élément de soupape (76) et un siège de soupape (78) montés dans ledit passage de distribution de matériau de revêtement (74), ledit élément de soupape (76) étant mobile par rapport audit siège de soupape (78) pour fournir sélectivement le matériau de revêtement à la chambre allongée (30).

8. Dispositif selon la revendication 7, dans lequel ladite structure de logement (12) comprend en outre un premier sous-logement (22) et un deuxième sous-logement (24), ledit premier sous-logement (22) étant couplé de manière amovible audit deuxième sous-logement (24) et contenant ladite chambre de revêtement allongée (30), et ledit deuxième sous-logement (24) contenant ledit élément de soupape (76) et ledit siège de soupape (78).

9. Dispositif selon la revendication 1, dans lequel l'objet allongé comprend en outre une aiguille creuse, et comprenant en outre :
un porte-aiguille (20) comprenant un espace d'air intérieur (20a) et un élément de support d'aiguille (40) configuré pour fixer une première extrémité (36a) de l'aiguille (36) en communication avec l'espace d'air intérieur (20a) alors qu'une deuxième extrémité ouverte (36b) de l'aiguille (36) s'étend vers l'extérieur dudit porte-aiguille (20) pour l'insertion à travers ledit orifice (35) et dans ladite chambre allongée (30), dans lequel ledit espace d'air intérieur (20a) est adapté pour être pressurisé avec de l'air pour forcer l'air à travers l'aiguille (36) et empêcher le colmatage de la deuxième extrémité ouverte (36b) de l'aiguille (36) avec le matériau de revêtement.

10. Dispositif selon la revendication 1, dans lequel ledit orifice (35) est configuré pour permettre à de l'air supplémentaire d'être aspiré dans ladite chambre allongée (30) à mesure que le brouillard revêt l'extérieur de l'objet allongé.

11. Procédé de revêtement d'une surface extérieure d'un objet allongé avec un matériau de revêtement en utilisant un dispositif selon la revendication 1, comprenant :
le maintien de l'objet allongé dans le sens de la longueur dans une chambre allongée (30) ayant des première et deuxième extrémités opposées (32, 34) et une sortie à la deuxième extrémité (34),
le mélange d'air comprimé et du matériau de revêtement pour former un brouillard ; et le revêtement de la surface extérieure avec le matériau de revêtement tout en dirigeant le brouillard autour de la surface extérieure et vers la sortie de la chambre allongée (30).

12. Procédé selon la revendication 11, comprenant en outre :
le mélange de l'air comprimé et du matériau de revêtement dans un passage de mélange (52) orienté transversalement à la chambre allongée (30) avant
l'introduction de l'air et du matériau de revêtement dans la chambre allongée (30),
dans lequel de préférence le passage de mélange (52) s'étend le long d'un axe, et le procédé comprend en outre :
l'introduction de l'air dans le passage de mélange (52) avec un effet Venturi.

13. Procédé selon la revendication 11, dans lequel le maintien de l'objet allongé comprend en outre l'introduction de l'objet à travers un orifice (35) communiquant avec la première extrémité (32) de la chambre allongée (30), et le procédé comprend en outre :
l'introduction d'air supplémentaire à travers l'orifice (35) et de manière adjacente à l'objet vers la sortie de la chambre allongée (30).

14. Procédé selon la revendication 11, dans lequel l'objet allongé comprend en outre une aiguille creuse avec des première et deuxième extrémités ouvertes opposées (36a, 36b), et le procédé comprend en outre :
la localisation de la deuxième extrémité ouverte (36b) dans la chambre allongée (30) ; et l'inroduction d'air à travers la première extrémité ouverte (36a) de l'aiguille (36) et hors de la deuxième extrémité ouverte (36b) pour empêcher le colmatage de la deuxième extrémité ouverte (36b) avec le matériau de revêtement.

15. Procédé selon la revendication 11, dans lequel l'introduction du matériau de revêtement comprend en outre :
l'actionnement d'une soupape pour permettre au matériau de revêtement comprimé de s'écouler dans la chambre allongée (30).

16. Procédé selon la revendication 11, dans lequel le matériau de revêtement comprend un matériau de réduction de friction.

17. Procédé selon la revendication 11, dans lequel l'introduction du brouillard vers la sortie comprend en outre :
l'introduction du brouillard à travers des sections de la chambre (30) ayant un diamètre croissant.
